# EUROPÄISCHE PATENTSCHRIFT

(11) **EP 0 720 512 B1**
(45) Veröffentlichungstag und Bekanntmachung des Hinweises auf die Patenterteilung: **29.12.1997**
(21) Anmeldenummer: 94927623.2
(22) Anmeldetag: 16.09.1994
(51) Int. Cl.: B01J 23/00, B01J 23/31, B01J 23/84, B01J 23/887

(54) **KATALYSATOR AUF DER BASIS VON Fe-, Co-, Bi- UND Mo-OXIDEN**
CATALYST BASED ON Fe, Co, Bi AND Mo OXIDES
CATALYSEURS A BASE D'OXYDES DE FER, DE COBALT, DE BISMUTH ET DE MOLYBDENE

(30) Priorität: 24.09.1993 DE 4332542
(43) Veröffentlichungstag der Anmeldung: 10.07.1996
(73) Patentinhaber: BASF AKTIENGESELLSCHAFT, 67056 Ludwigshafen (DE)
(72) Erfinder: KARRER, Lothar, D-64319 Pfungstadt (DE); NEUMANN, Hans-Peter, D-68307 Mannheim (DE); EICHHORN, Hans-Dieter, D-67273 Weisenheim am Berg (DE); JARRET, Robin, Stuart, Yarm, Cleveland TS15 9QA (GB)
(86) Internationale Anmeldenummer: EP9403111
(87) Internationale Veröffentlichungsnummer: WO9508391

(56) Entgegenhaltungen:
- EP-A- 0 000 835
- EP-A- 0 342 777
- EP-A- 0 344 844
- EP-A- 0 352 023
- EP-A- 0 575 897
- US-A- 4 388 223

## Beschreibung

Die vorliegende Erfindung betrifft Katalysatoren der allgemeinen Formel I

[AₐB_{b}Oₓ]ₚ [C_{c}D_{d}FeₑCo_{f}EᵢFⱼO_{y}]_{q} I,

in der die Variablen folgende Bedeutung haben:
A Bismut, Tellur, Antimon, Zinn und/oder Kupfer,
B Molybdän und/oder Wolfram,
C ein Alkalimetall, Thallium und/oder Samarium,
D ein Erdalkalimetall, Nickel, Kupfer, Kobalt, Mangan, Zink, Zinn, Cer, Chrom, Cadmium, Molybdän, Bismut und/oder Quecksilber,
E Phosphor, Arsen, Bor und/oder Antimon,
F ein Seltenerdmetall, Vanadium und/oder Uran,
a 0,01 bis 8,
b 0,1 bis 30,
c 0 bis 4,
d 0 bis 20,
e 0 bis 20,
f 0 bis 20,
i 0 bis 6,
j 0 bis 15,
x, y Zahlen, die durch die Wertigkeit und Häufigkeit der von Sauerstoff verschiedenen Elemente in I bestimmt werden und
p,q Zahlen, deren Verhältnis p/q im Bereich von 0,001 bis 0,099 liegt.

Außerdem betrifft die Erfindung ein Verfahren zur Herstellung dieser Katalysatoren, deren Verwendung zur Ammonoxidation, Oxidation oder Dehydrierung ethylenisch ungesättigter Verbindungen sowie ein Verfahren zur Ammonoxidation, Oxidation oder oxidativen Dehydrierung ethylenisch ungesättigter Verbindungen in Gegenwart der erfindungsgemäßen Katalysatoren.

Die EP-A-000 835 beschreibt Katalysatoren mit der Zusammensetzung

[M'_{m'}N'_{n'}O'_{x'}]_{q'} [A'_{a'}C'_{b'}D'_{c'}E'_{d'}F'_{e'}N'_{f'}O'_{y'}]_{p'},

wobei
M' = Bi, Te, Sb, Sn und/oder Cu
N' = Mo und/oder W
A' = Alkalimetall, Tl und/oder Sm
C' = Ni, Co, Mn, Mg, Be, Ca, Sr, Ba, Zn, Cd und/oder Hg
D' = Fe, Cr, Ce und/oder V
E' = P, As, B, Sb
F' = Seltenerdmetall, Ti, Zr, Nb, Ta, Re, Ru, Rh, Ag, Au, Al, Ga, In, Si, Ge, Pb, Th und/oder U
a' = 0-4
b' = 0-20
c' = 0,01-20
d' = 0-4
e' = 0-8
f' = 8-16
m' = 0,01-10
n' = 0,1-30, bedeuten,
und x' und y' Zahlen sind, die durch die Wertigkeit und Häufigkeit der von Sauerstoff verschiedenen Elemente bestimmt werden, und
q' und p' Zahlen sind, deren Verhältnis q'/p' im Bereich von 0,1 bis 10 liegt.

Die Komponente [M'_{m'}N'_{n'}O'_{x'}] wird als Schlüsselphase, die Komponente [A'_{a'}C'_{b'}D'_{c'}E'_{d'}F'_{e'}N'_{f'}O'_{y'}] als Gastphase bezeichnet.

Hinsichtlich der Herstellung dieser Katalysatoren empfiehlt die EP-A 000 835 die Komponente [M'_{m'}N'_{n'}O'_{x'}] in Abwesenheit der übrigen Konstituenten vorzubilden, und sie nach ihrer Vorbildung mit Oxiden oder wasserlöslichen Salzen der Elemente der Gastphase zu vermischen und das Gemisch nach Trocknung zu calcinieren.

Weiterhin ist aus der EP-A 000 835 bekannt, die dort genannten katalytischen Massen als Katalysatoren für gasphasenkatalytische Oxidationen organischer Verbindungen einzusetzen. Nachteile an den in der EP-A 000 835 offenbarten Massen ist jedoch, daß sie bei Anwendung in gasphasenkatalytischen Oxidationen organischer Verbindungen sowohl hinsichtlich Aktivität als auch Selektivität nicht voll zu befriedigen vermögen.

Aus der DE-C 33 38 380 sind katalytisch aktive Massen der allgemeinen Formel II

Bi_{a''}W_{b''}Fe_{c''}Mo_{d''}Y¹_{e''}Y²_{f''}Y³_{g''}Y⁴_{h''}O_{x''} (II),

in der die Variablen folgende Bedeutung haben:
Y¹ Nickel und/oder Kobalt
Y² Thallium, ein Alkalimetall und/oder ein Erdalkalimetall,
Y³ Phosphor, Arsen, Bor, Antimon, Zinn, Cer, Blei und/oder Niob,
Y⁴ Silicium, Aluminium, Zirkonium und/oder Titan,
d' 12, 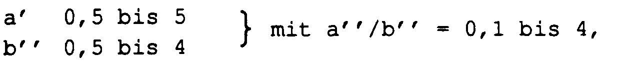
c'' 0,2 bis 5,
e'' 3 bis 10,
f'' 0,02 bis 2,
g'' 0 bis 5,
h'' 0 bis 10 und
x'' Zahl, die durch die Wertigkeit und Häufigkeit der von Sauerstoff verschiedenen Elemente in II bestimmt wird,
bekannt, die dadurch erhalten werden, daß man zunächst eine Wismut- und eine Wolframverbindung in wäßrigem Medium vermischt, das wäßrige Gemisch trocknet, die resultierende Masse bei 600 bis 900°C calciniert und anschließend so pulverisiert, daß die Korngröße weniger als 152 µm beträgt, sowie das dabei erhaltene Pulver mit einer wäßrigen Lösung der Quellen der übrigen Konstituenten der Masse II versetzt, die resultierende Mischung einengt, formt und calciniert.

Weiterhin ist aus der DE-C 33 38 380 bekannt, daß die Massen II als Katalysatoren für die gasphasenkatalytisch-oxidative Herstellung ungesättigter Aldehyde geeignet sind.

Nachteilig an den in der DE-C 33 38 380 offenbarten Massen II ist jedoch, daß sie bei der Ammoxidation von Propen zu Acrylnitril sowohl hinsichtlich Aktivität als auch Selektivität nicht voll zu befriedigen vermögen.

Aufgabe der vorliegenden Erfindung war es daher, Katalysatoren zur Verfügung zu stellen, die die genannten Nachteile nicht aufweisen.

Demgemäß wurden die in Anspruch 1 definierten Katalysatoren gefunden.

Als Elemente der erfindungsgemäßen Katalysatoren kommen in Betracht:
A Bismut, Tellur, Antimon, Zinn und/oder Kupfer,
B Molybdän und/oder Wolfram,
C ein Alkalimetall, Thallium und/oder Samarium,
D ein Erdalkalimetall, Nickel, Kupfer, Kobalt, Mangan, Zink, Zinn, Cer, Chrom, Cadmium, Molybdän, Bismut und/oder Quecksilber,
E Phosphor, Arsen, Bor und/oder Antimon,
F ein Seltenerdmetall, Vanadium und/oder Uran,
a 0,01 bis 8, bevorzugt 0,1 bis 6,
b 0,1 bis 30, bevorzugt 0,2 bis 8,
c 0 bis 4, bevorzugt 0,01 bis 3,
d 0 bis 20, bevorzugt 0 bis 15,
e 0 bis 20, bevorzugt 0 bis 15,
f 0 bis 20, bevorzugt 0 bis 15,
i 0 bis 6, bevorzugt 0 bis 4,
j 0 bis 15, bevorzugt 0 bis 10.

Solche Katalysatoren sind bevorzugt, welche wenigstens eines der Elemente Bismut, Molybdän und/oder Wolfram und Eisen mit einem von Null verschiedenen stöchiometrischen Koeffizienten enthalten, der vorzugsweise ≥ 0,01 beträgt.

Ferner erweisen sich die erfindungsgemäßen Katalysatoren I als umso vorteilhafter, je größer der prozentuale Anteil der verschiedenen chemisch unterschiedlichen Komponenten mit einer mittleren Korngröße (gemessen mit Sympatec Laserbeugungsgerät) im Bereich von 10 nm bis kleiner als 1 µm, vorzugsweise im Bereich von 10 nm bis 800 nm, besonders bevorzugt von 10 nm bis 600 nm, ist.

Insbesondere haben Teilchen der Schlüsselphase und/oder der Gastphase des Katalysators bevorzugt eine mittlere Korngröße im Bereich von 10 nm bis kleiner als 1 µm, besonders bevorzugt im Bereich von 10 nm bis 800 nm, ganz besonders bevorzugt von 10 nm bis 600 nm.

Dabei handelt es sich bei der Schlüsselphase AₐB_{b}Oₓ vorzugsweise um solche der Stöchiometrie

Bi₂W₂0₉ und/oder Bi₂Mo₂0₉,

unter denen das Bi₂W₂0₉ bevorzugt ist.

Die erfindungsgemäßen Massen sind durch folgende Schritte erhältlich:
(a) Herstellen einer Schlüsselphase mit der Zusammensetzung AₐB_{b}Oₓ,
(b) Herstellen einer Gastphase der Zusammensetzung C_{c}D_{d}FeₑCo_{f}EᵢFⱼO_{y} oder wasserlöslichen Salzen der Elemente der Gastphase oder einer Mischung aus mindestens einem wasserlöslichen Salz dieser Elemente und mindestens einer calcinierten Phase eines dieser Elemente, das nicht als wasserlösliches Salz vorliegt und
(c) Mischen der Schlüsselphase mit der Gastphase und
(d) gewünschtenfalls Trocknen der nach (c) erhaltenen Mischung, anschließendes Calcinieren und gewünschtenfalls Formen der so gebildeten Katalysatormasse in an sich bekannter Weise (s. EP-A 000 835, DE-C 3,338,380).

In einer besonderen Ausführungsform zerkleinert man mindestens eine der Katalysatorkomponenten (Schlüsselphase, deren Vorstufe, Gastphase oder eine calcinierte Oxidphase eines der in der Gastphase vorkommenden Verbindungen) mit an sich bekannten Mitteln, beispielsweise einer Kugelmühle oder durch Strahlmahlen, wobei eine Naßzerkleinerung bevorzugt ist. Die durch die Zerkleinerung erreichte mittlere Korngröße wählt man bevorzugt im Bereich von 10 nm bis kleiner als 1 µm, besonders bevorzugt von 10 nm bis 800 nm, ganz besonders bevorzugt von 10 nm bis 600 nm.

Die zerkleinerte Katalysatorkomponente mischt man dann üblicherweise mit den restlichen Katalysatorkomponenten, gewünschtenfalls ebenfalls zerkleinert, bevorzugt in Lösung oder Suspension.

Die erhaltene Mischung wird im allgemeinen einer Trocknung, vorzugsweise einer Sprühtrocknung unter Erhalt eines Sprühguts, unterworfen.

Das so erhaltene Katalysatorvorprodukt wird dann in der Regel bei Temperaturen im Bereich von 400 bis 900°C, bevorzugt von 500 bis 800°C, vorzugsweise im Luftstrom calciniert. Die Calcinierungsdauer wählt man in der Regel im Bereich von 0,1 bis 20 h.

Bevorzugt bringt man die katalytisch aktive Masse auf einem oxidischen Träger wie SiO₂, Al₂O₃, TiO₂ oder ZrO₂ auf. In einer bevorzugten Ausführungsform mischt man vor dem Sprühtrocknen den oxidischen Träger wie SiO₂, Al₂O₃, TiO₂ oder ZrO₂ der Mischung der Katalysatorkomponenten bei, wobei man den so hergestellten Katalysator direkt in einem Wirbelschichtreaktor für organische Synthesen einsetzen kann.

Des weiteren kann man den calcinierten Katalysator in an sich bekannter Weise zerkleinern und formen, z.B. indem man nach an sich bekannten Methoden Hohlzylinder oder Stränge preßt.

In einer weiteren bevorzugten Ausführungsform zerkleinert man das Vorprodukt der Schlüsselphase, [AₐB_{b}Oₓ], wobei man das Vorprodukt der Schlüsselphase vor dem Zerkleinern vorzugsweise bei einer Temperatur im Bereich von 400 bis 900°C üblicherweise im Luftstrom calciniert. Die Calcinierungsdauer wählt man in der Regel im Bereich von 0,1 bis 20 h.

Von den übrigen Bestandteilen des gewünschten erfindungsgemäßen Katalysators wird im allgemeinen ausgehend von in an sich bekannter Weise geeigneten Quellen (vgl. EP-A 835 und DE-C 33 38 380) ein möglichst inniges, vorzugsweise feinteiliges, Trockengemisch hergestellt (z.B. wasserlösliche Salze wie Halogenide, Nitrate, Acetate, Carbonate oder Hydroxide in einer wäßrigen Lösung vereinen und anschließend die wäßrige Lösung Sprühtrocknen oder nicht wasserlösliche Salze, z.B. Oxide, in wäßrigem Medium suspendieren und anschließend die Suspension sprühtrocknen), das hier als Vorprodukt der Gastphase bezeichnet wird. Wesentlich ist nur, daß es sich bei den Bestandteilen der Vorgastphase entweder bereits um Oxide handelt, oder um solche Verbindungen, die durch Erhitzen, gegebenenfalls in Anwesenheit von Sauerstoff, in Oxide überführbar sind.

Anschließend werden üblicherweise das calcinierte Vorprodukt der Schlüsselphase und das Vorprodukt der Gastphase im gewünschten Mengenverhältnis miteinander vermischt, vorzugsweise durch Pressen verdichtet, und danach (normalerweise im Luftstrom) zweckmäßig bei Temperaturen von 400 bis 900°C, mehrere Stunden calciniert.

Im Falle von Vollkatalysatoren erfolgt das Verpressen in der Regel unmittelbar zur gewünschten Katalysatorgeometrie, wobei als solche Hohlzylinder mit einem Außendurchmesser und einer Länge von 2 bis 10 mm und einer Wandstärke von 1 bis 3 mm bevorzugt werden. Die aktiven erfindungsgemäßen Katalysatoren können nach dem Calcinieren aber auch zerkleinert und auf inerte Träger zur Herstellung von Trägerkatalysatoren aufgebracht werden. Das Aufbringen kann auch bereits vor der abschließenden Calcinierung erfolgen. In diesem Fall erfolgt das Aufbringen vorzugsweise gemäß der EP-B 293 859. Selbstverständlich können die erfindungsgemäßen Massen auch in Pulverform eingesetzt werden.

Die erfindungsgemäßen Katalysatoren weisen gegenüber entsprechenden Katalysatoren des Standes der Technik sowohl eine erhöhte Aktivität als auch eine erhöhte Selektivität für gasphasenkatalytische Oxidationen organischer Verbindungen wie niederer (3 bis 6 C-Atome) Alkane, Alkanole, Alkanale, Alkene und Alkenale zu olefinisch ungesättigten Aldehyden und/oder Carbonsäuren sowie zu den entsprechenden Nitrilen (Ammonoxidation) auf, vor allem von Propen zu Acrylnitril und von i-Buten bzw. tert.-Butanol zu Methacrylnitril. Des weiteren eignen sie sich zur oxidativen Dehydrierung organischer Verbindungen.

### Beispiele

### Beispiel 1

(a) Herstellung einer Schlüsselphase
   0,5 kg einer Lösung von Bi(NO₃)₃ in wäßriger Salpetersäure (11 Gew.-% Bi, 6,4 Gew.-% HNO₃, jeweils auf die Lösung bezogen) wurden mit 67 g H₂WO₄ versetzt und 1 h bei 50°C gerührt.
   Die erhaltene Suspension wurde bei 290°C sprühgetrocknet und 2 h bei 750°C calciniert. Das so erhaltene vorgebildete calcinierte Mischoxid (Bi₂W₂O₉) mit geringer WO₃-Verunreinigung wurde anschließend mit einer Rührwerksmühle auf eine mittlere Korngröße von 400 nm naßzerkleinert (gemessen an einem Sympatec-Laserbeugungsgerät), wobei die Suspension 1 erhalten wurde (Vorprodukt Schlüsselphase 1).
(b) Herstellung einer Gastphase
   Eine Lösung von 5,57 kg Ammoniumheptamolybdat in 16 l Wasser wurde mit einer Lösung, die 3,83 kg Kobalt(II)nitrat und 2,66 kg Eisen(III)nitrat in 8 l 10 gew.-%iger Salpetersäure gelöst enthielt sowie mit 19,1 kg einer 49 % ihres Gewichtes kolloidales SiO₂ enthaltenden wäßrigen Mischung und 15,4 g einer 48 Gew.-% KOH enthaltenden wäßrigen Lösung versetzt (Suspension 2).
   Die Suspension 1 wurde anschließend mit der Suspension 2 gemischt. Die Mischung wurde dann durch Sprühtrocknung zur Trockene eingedampft und anschließend bei 290°C für 3 h, dann bei 425°C für weitere 3 h und schließlich bei 610°C für weitere 3 h calciniert. Der so erhaltene Katalysator konnte direkt in einem Wirbelschichtreaktor eingesetzt werden.
   Der Katalysator hatte folgende Zusammensetzung: [Bi₂W₂O₉]_{0,05}[Mo₁₂Co₅Fe_{2,5}K_{0,05}Oₓ]

### Beispiel 2 - Vergleichskatalysator (analog zu Beispiel 1 aus EP-A 000 835)

Beispiel 1 wurde wiederholt, mit dem Unterschied allerdings, daß das Vorprodukt der Schlüsselphase direkt nach Sprühtrocknung ohne vorherige Zerkleinerung eingesetzt wurde.

### Beispiel 3

### Ammonoxidation von Propen

(a) Bei einer Temperatur von 450°C wurde ein Gasgemisch aus Propen/Ammoniak/Luft/Wasser mit dem Volumenverhältnis 1/1,3/10/4,7 in einem Festbettreaktor zur Reaktion gebracht. Die Kontaktzeit (Volumen(Gas)/Volumen(Katalysator) pro Zeiteinheit) betrug 4,5 sec. Als Katalysator wurde der Katalysator aus Beispiel 1 eingesetzt.
(b) Beispiel 3(a) wurde mit dem mit Katalysator aus Beispiel 2 wiederholt.

Die Ergebnisse der Ammonoxidation finden sich in der nachfolgenden Tabelle.

**Tabelle**

| Katalysator | Propenumwandlung [%] | Ausbeute [Mol-%] | Selektivität [%] |
|---|---|---|---|
| Beispiel 1 | 98,5 | 85,8 | 87,1 |
| zum Vergleich Beispiel 2 | 98,1 | 82,2 | 83,8 |

## Patentansprüche

1. Katalysator der allgemeinen Formel I
[AₐB_{b}Oₓ]ₚ [C_{c}D_{d}FeₑCo_{f}EᵢFⱼO_{y}]_{q} I,
in der die Variablen folgende Bedeutung haben:
A Bismut, Tellur, Antimon, Zinn und/oder Kupfer,
B Molybdän und/oder Wolfram,
C ein Alkalimetall, Thallium und/oder Samarium,
D ein Erdalkalimetall, Nickel, Kupfer, Kobalt, Mangan, Zink, Zinn, Cer, Chrom, Cadmium, Molybdän, Bismut und/oder Quecksilber,
E Phosphor, Arsen, Bor und/oder Antimon,
F ein Seltenerdmetall, Vanadium und/oder Uran,
a 0,01 bis 8,
b 0,1 bis 30,
c 0 bis 4,
d 0 bis 20,
e 0 bis 20,
f 0 bis 20,
i 0 bis 6,
j 0 bis 15,
x, y Zahlen, die durch die Wertigkeit und Häufigkeit der von Sauerstoff verschiedenen Elemente in I bestimmt werden und
p,q Zahlen, deren Verhältnis p/q im Bereich von 0,001 bis 0,099 liegt,
erhältlich durch folgende Schritte;
(a) Herstellen einer Schlüsselphase mit der Zusammensetzung AₐB_{b}Oₓ,
(b) Herstellen einer Gastphase der Zusammensetzung C_{c}D_{d}FeₑCo_{f}EᵢFⱼO_{y} oder wasserlöslichen Salzen der Elemente der Gastphase oder einer Mischung aus mindestens einem wasserlöslichen Salz dieser Elemente und mindestens einer calcinierten Phase eines dieser Elemente, das nicht als wasserlösliches Salz vorliegt und
(c) Mischen der Schlüsselphase mit der Gastphase und
(d) gewünschtenfalls Trocknen der nach (c) erhaltenen Mischung, anschließendes Calcinieren und gewünschtenfalls Formen der so gebildeten Katalysatormasse in an sich bekannter Weise,
wobei die
mittlere Korngröße mindestens einer den Katalysator aufbauenden Komponente, die sich in ihrer chemischen Zusammensetzung von den anderen Komponenten unterscheidet, im Bereich von 10 nm bis kleiner als 1 µm liegt.

2. Katalysator gemäß Anspruch 1, dadurch gekennzeichnet, daß der Katalysator auf einem oxidischen Trägermaterial aufgebracht ist.

3. Verfahren zur Herstellung eines Katalysators gemäß den Ansprüchen 1 bis 2 durch Herstellen einer Schlüssel- und einer Gastphase, Mischen der beiden Phasen und anschließendes Calcinieren der Mischung dieser Phasen, dadurch gekennzeichnet, daß die Schlüsselphase die Zusammensetzung AₐB_{b}Oₓ und die Gastphase die Zusammensetzung C_{c}D_{d}FeₑCo_{f}EᵢFⱼO_{y} hat.

4. Verwendung des Katalysators gemäß den Ansprüchen 1 bis 2 zur Ammonoxidation, Oxidation oder oxidativen Dehydrierung ethylenisch ungesättigter Verbindungen.

5. Verfahren zur Ammonoxidation, Oxidation oder oxidativen Dehydrierung ethylenisch ungesättigter Verbindungen in an sich bekannter Weise, dadurch gekennzeichnet, daß man das Verfahren in Gegenwart eines Katalysators gemäß der Ansprüche 1 bis 2 einsetzt.

## Claims

1. A catalyst of the formula I
[AₐB_{b}Oₓ]ₚ [C_{c}D_{d}FeₑCo_{f}EᵢFⱼO_{y}]_{q} I,
where
A is bismuth, tellurium, antimony, tin and/or copper,
B is molybdenum and/or tungsten,
C is an alkali metal, thallium and/or samarium,
D is an alkaline earth metal, nickel, copper, cobalt, manganese, zinc, tin, cerium, chromium, cadmium, molybdenum, bismuth and/or mercury,
E is phosphorus, arsenic, boron and/or antimony,
F is a rare-earth metal, vanadium and/or uranium,
a is from 0.01 to 8,
b is from 0.1 to 30,
c is from 0 to 4,
d is from 0 to 20,
e is from 0 to 20,
f is from 0 to 20,
i is from 0 to 6,
j is from 0 to 15,
x and y are numbers determined by the valency and frequency of the elements other than oxygen in I, and
p and q are numbers whose ratio p/q is in the range from 0.001 to 0.099,
obtainable by the following steps:
(a) preparation of a key phase having the composition AₐB_{b}Oₓ,
(b) preparation of a guest phase having the composition C_{c}D_{d}FeₑCo_{f}EᵢFⱼO_{y} or water-soluble salts of elements of the guest phase or a mixture of at least one water-soluble salt of these elements and at least one calcined phase of one of these elements which is not in the form of a water-soluble salt, and
(c) mixing of the key phase with the guest phase, and
(d) if desired drying of the mixture obtained in (c), followed by calcination and, if desired, shaping of the catalyst composition formed in this way in a manner known per se,
the mean particle size of at least one component making up the catalyst, which differs in chemical composition from the other components, being in the range from 10 nm to less than 1 µm.

2. A catalyst as claimed in claim 1, wherein the catalyst has been applied to an oxidic support material.

3. A process for the preparation of a catalyst as claimed in claim 1 or 2 by preparing a key phase and a guest phase, mixing the two phases and subsequently calcining the mixture of these phases, wherein the key phase has the composition AₐB_{b}Oₓ and the guest phase has the composition C_{c}D_{d}FeₑCo_{f}EᵢFⱼO_{y}.

4. The use of a catalyst as claimed in claim 1 or 2 for the ammonoxidation, oxidation or oxidative dehydrogenation of ethylenically unsaturated compounds.

5. A process for the ammonoxidation, oxidation or oxidative dehydrogenation of ethylenically unsaturated compounds in a manner known per se, which comprises commencing the process in the presence of a catalyst as claimed in claim 1 or 2.

## Revendications

1. Catalyseur de la formule générale I
[AₐB_{b}Oₓ]ₚ [C_{c}D_{d} FeₑCo_{f}EᵢFⱼO_{y}]_{q} I,
dans laquelle les diverses variables ont les significations qui suivent :
A bismuth, tellure, antimoine, étain et/ou cuivre,
B molybdène et/ou tungstène,
C un métal alcalin, thallium et/ou samarium,
D un métal alcalino-terreux, nickel, cuivre, cobalt, manganèse, zinc, étain, cérium, chrome, cadmium, molybdène, bismuth et/ou mercure,
E phosphore, arsenic, bore et/ou antimoine,
F un métal des terres rares, vanadium et/ou uranium,
a 0,01 à 8,
b 0,1 à 30,
c 0 à 4,
d 0 à 20,
e 0 à 20,
f 0 à 20,
i 0 à 6,
j 0 à 15,
x, y des nombres qui sont déterminés par la valence et la fréquence des éléments qui diffèrent de l'oxygène dans I et p, q des nombres dont le rapport p/q varie dans la plage de 0,001 à 0,099,
que l'on peut obtenir par les étapes suivantes :
(a) Préparation d'une phase clé de la composition AₐB_{b}Oₓ,
(b) Préparation d'une phase hôte de la composition C_{c}D_{d} FeₑCo_{f}EᵢFⱼO_{y} ou des sels hydrosolubles des éléments de la phase hôte ou d'un mélange d'au moins un sel hydrosoluble de ces éléments et d'au moins une phase calcinée d'un de ces éléments, qui ne se présente pas sous forme de sel hydrosoluble et
(c) Mélange de la phase clé à la phase hôte et
(d) Si on le souhaite, séchage du mélange obtenu selon (c), calcination subséquente et moulage éventuel de la masse de catalyseur ainsi formée, de manière en soi connue,
où
la granulométrie moyenne d'au moins l'un des composant constitutifs du catalyseur, qui se différencie des autres composants par sa composition chimique, se situe dans la plage de 10 nm à moins de 1 µm.

2. Catalyseur suivant la revendication 1, caractérisé en ce que le catalyseur est appliqué sur un matériau de support oxydique.

3. Procédé de préparation d'un catalyseur suivant les revendications 1 et 2, par la préparation d'une phase clé et d'une phase hôte, mélange des deux phases et calcination subséquente du mélange de ces phases, caractérisé en ce que la phase clé possède la composition AₐB_{b}Oₓ, et la phase hôte possède la composition C_{c}D_{d}FeₑCo_{f}EᵢFⱼO_{y}.

4. Utilisation du catalyseur suivant les revendications 1 et 2, pour la monoxydation, l'oxydation ou la déshydrogénation oxydante de composés à insaturation éthylénique.

5. Procédé d'ammonoxydation, d'oxydation ou de déshydrogénation oxydante de composés à insaturation oléfinique, d'une manière en soi connue, caractérisé en ce que l'on entreprend le procédé en présence d'un catalyseur suivant les revendications 1 et 2.
